# EUROPEAN PATENT APPLICATION

(11) **EP 1 625 844 A1**
(43) Date of publication of application: **15.02.2006**
(21) Application number: 03727132.7
(22) Date of filing: 16.05.2003
(51) Int. Cl.: A61K 9/00, A61K 31/015, A61K 31/355, A61K 31/375, A61K 31/455, A61K 31/525, A61K 31/4415, A61P 17/10

(54) **THE COMPOSITION FOR EXTERNAL USE BY PERCUTANEOUS ADMINISTRATION**

(71) Applicant: Lotus Pharmaceutical Co., Ltd., Da-an Chiu, Taipei City 106 (TW)
(72) Inventor: LIU, Busang, Sanchung City, Taipei County 241 (CN); LIN, Tongho, Da-an Chiu, Taipei City 106 (CN)
(74) Representative: Zimmermann, Tankred Klaus
(86) International application number: PCT/CN2003/000358
(87) International publication number: WO 2004/100923

(57) **Abstract**

The present invention relates to a topical composition for transdermal administration, wherein the composition mainly comprises vitamin C, vitamin B complex, carotene, vitamin E and flavor, thickening agent and surfactant. Said pharmaceutical composition is locally applicable and useful in skin-care, and can be used for the treatment of acne, comedo and zit, and also has the function ofantioxidation.

## Description

### BACKGROUND OF THE INVENTION

### Field of the Invention

The present invention relates to the topical composition for transdermal administration. The typical pharmaceutical composition can be used for nourishing and improving skin and treating acne, comedo and zit. Said composition has an antioxidation property and is free of Vitamin A Acid.

### Description of the Prior Art

The skin construction of human being consists of epidermis, dermis, subcutaneous fatty tissue, sebaceous gland, sweat glands, hairs and nail. The thick layer under epidermis is named as dermis. The hairs are surrounded by the hair follicle, and there is sebaceous gland by its side. The sebaceous gland can secrete sebum, the sebum will permeate through the surface of skin by the hairs and hair follicle, and then convert into fatty film, which will attach to the surface of skin and protect the skin. The type of skin is classified according to the quantity of sebaceous gland, and comprises oily skin, dry-type skin and mixed-type skin.

Acne is a chronic inflammation in the hair follicle and sebaceous gland, which is also commonly named as comedo or zit. It is usually can be found on the forehead, around the nosewing, on the cheeks, and it can also can be found on any part of the body with hair follicle, such as the back, thoracic part and thigh. Depending on its symptoms, it can be classified as acne-type, Inflammatory and red swelling type, and cyst-type. When the gonad of youth became mature, the content of androgenic hormone in the testes and Ovaries will increase, the androgenic hormone will stimulate the sebaceous gland, and then the sebaceous gland will became hypertrophy and secrete abundance sebum which will stack around the sebaceous gland and hair follicle, then get inflamed and affected by the bacterium. Usually, an acne with white or black head is formed at first, subsequently become papule, pustule, node and cyst after it is infected with bacterium and then a zit is formed. When too much androgenic hormone is secreted, red, tickle, heat, burning and peel will appear around the T area of face or the skin under the eyelids, and sometimes will appear visible microvessels, which is named as seborrheic dermatitis.

To solve these problems, The topical preparations are needed to clean the skin, kill the bacterium on the skin, prevent water evaporating from skin and improve the moisture of skin, e.g. the a cream containing sodium chloride for mollifying and soothing to the skin disclosed in US patent No.3,574,854, a skin-care composition containing mineral salts disclosed in DE patent publication No.3,327,840. US patent No.3,859,436 disclosed glucose mixture for soothing skin, US patent No.3,777,597 disclosed a shaving aqueous solution comprising glucose.

Sodium Chloride, especially 0.9% sodium chloride solution i.e. saline, is the main composition to maintain the osmotic pressure of body fluid, but the sodium chloride with higher concentration can not be absorbed by the skin, it will irritate the skin mucosa and thus result in dehydration; The topical preparations containing sodium chloride may stimulate skin or kill bacterium, but it can hardly prevents water evaporating from skin and it can hardly improves the moisture of skin. Although glucose may increase the glycogen, ensure all the cells hyperfunction, improve the organism metabolism function as a nutritional agent and has the function of detoxication, but it is difficult to externalize the above-described specific effects by applying the topical preparations containing glucose to skin, unless the glucose is administered orally, intravenous and intramuscular injection.

There have been many therapeutic agents and cosmetics for treating these skin indications, such as Hydrocortisone for treating the atopic dermatitis with titillate and erythema, sulconazole nitrate for treating mycotic infection in the skin, tretinoin for treating light aging, and 5-fluorouracil for treating psoriasis and skin cancer. Generally, It is necessary to add permeation enhancers to the medicines for used in treatirig dermatogic disease, such as dimethyl sulfoxide(DMSO), dimethyl formamide, methyldecyl sulfoxide(US Patent No.3,527,864), dimethyl acetamide(US Patent No.3,472,931) and N-alkyl-2-pyrrolidone(US Patent No.3,696,516). However, the above-described permeation enhancers have certain disadvantages, for example, dimethyl sulfoxide has some odours and body odour, it can burn the skin, and induce erythema on skin, reduce the transparence of crystalline-skin and even cause tissue necrosis in the animals. (Martindale, The Extra Pharmacopoeia, pages 1461-1463, 27^{th} Ed., 1977). Dimethyl formamide and dimethyl acetamide can also bum the skin and induce erythema on skin.

Moreover, Trebosc, etc.(US patent No.5,030,451)disclosed a cosmetics composition containing modified derivatives of caffeine as active principle , the formulation have excellent and long-acting "lipolytic" properties and have therefore been proven very effective in slenderizing programs and in the treatment of cellulitis. An anti-cellulite composition is provided by Mausner in US patent No.5,215,759 using methylsilanol theophyllin acetate alginate and methylsilanol mannuronate. In US patent No.5,051,449, Kligman disclosed a method of prevent cellulite by locally applying to skin a retinoid, the treated subjects have a thickened epidermis and an increased number of new blood vessels, and an amelioration from moderate to noticed degree can observed in the pinching test.

Topical aminolevulinic acid-photodynamic therapy for the treatment of acne vulgaris is disclosed in US patent application No.20020099094. US patent application No.20020061855A disclosed a composition for treating acne comprising water and glycol, US patent application No.20010056071 disclosed a composition for treating comprising resveratrol (3,4',5-trihydroxy-trans-stilbene), melatonin, Vitamins D, E, and A.

In addition, Shapiro SS and Saliou C. described that vitamins A, vitamins D and their derivatives in combination with vitamins C, vitamins E, and coenzyme Q could improve the skin and cure acne(*Nutrition* 2001, Vol 17(10), page 839-844).Vitamin A Acid Acid( i.e.Tretinoin or. Retinoids) belongs to the derivatives of Vitamin A Acid according to its structure, and its major function is to remove cutin, because it can remove the cutin on the epiderm, improve the occlusions of pore, improve the wrinkle of skin and blood circulation around the face, decrease the scar of pigment and prevent the keratinization of skin, promote the refreshing and sloughing off of epithelium cells, prevent synthesis of keratinization, and prevent the formation of blister on the face. However, mostVitamin A Acid-containing products may make the skin sensitive to the light, and will cause skin's side effects, such as dry, red swelling, itching and dermatitis etc.

In conclusion, from the foregoing, although there are certain patents related to the therapy of acne, decomposing fat or various skin symptoms, all these patents have some defects and may do harm to the skin. Accordingly, the present invention provides a Vitamin A Acid acid-free topical composition for transdermal administration.

### SUMMARY OF THE INVENTION

The objective of this invention is to provide a topical composition for transdermal administration, said composition is free of Vitamin A Acid and mainly used for skin - care, threating acne and zit, and has an antioxidation property.

The invention provides a topical composition for transdermal administration, which mainly comprise vitamin C 1~45% W/W, vitamin B complex 1~5%W/W, carotene 1~3%W/W, and vitamin E 2~90% W/W.

The invention provides a topical composition for transdermal administration, mainly comprising vitamin C 4~15% W/W, vitamin B complex 1~3%W/W, carotene 1~2%W/W, vitamin E 20~65% W/W; [further comprising] Flavor 0.1~2% W/W; thickening agent 1~5% W/W, surfactants 1~8% W/W and appropriate amount of distilled water.

The above-described topical composition is mainly applied to local area of the skin.

The present invention also provides a topical composition for transdermal administration, which is mainly used for the manufacture of medicine useful in treatment of acne, comedo and zit, said composition also used for nourishing skin and has an antioxidation property. The composition mainly comprise vitamin C 1~45% W/W, vitamin B complex 1~5%W/W, carotene 1~3%W/W, vitamin E 2-90% W/W, Flavor 0.1~2% W/W, thickening agent 1-5% W/W, surfactants 1~8% W/W and appropriate amount of distilled water.

This invention provides a topical composition for transdermal administration, which mainly comprise vitamin C 1-45% W/W, vitamin B complex 1~5%W/W, carotene 1~3%W/W, vitamin E 2-90% W/W, Flavor 0.1~2% W/W, thickening agent 1-5% W/W, surfactants 1-8% W/W and distilled water, the sum of the content of the components is 100%.

The invention provides a topical composition for transdermal administration, useful in treatment and/or prevention acne, comedo and zit of the skin.

The present invention provides a composition for skin-care, which can also be used to improve the acne, comedo and zit of the skin, and has the function of anti-oxidation. As the composition is free of Vitamin A Acid, it will not induce the skin sensitive to light, and will improve symptoms of the skin without skin dry, red swelling, itching and dermatitis. There is no overdosage problem even applied over a long term.

By reference to the following drawings, the following non-limiting examples are illustrative of the present invention.

### BRIEF DESCRIPTION OF THE DRAWINGS

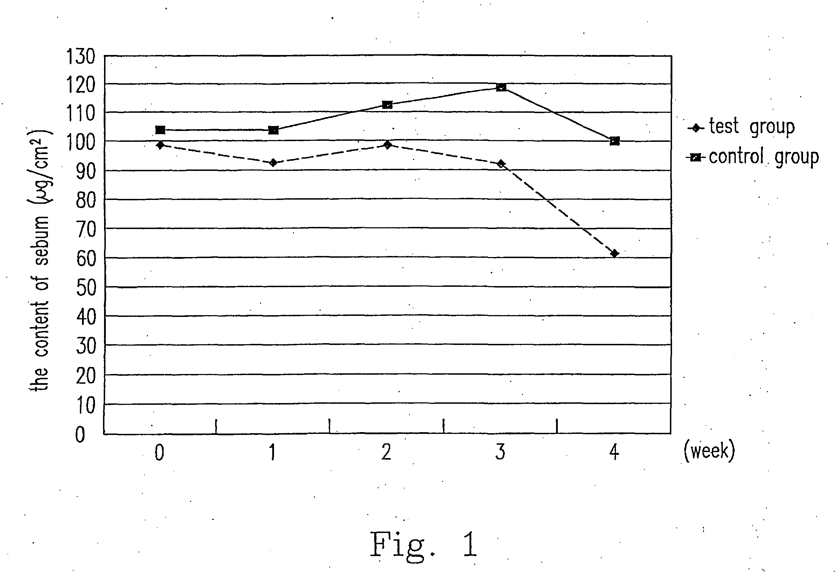

Fig.1 shows the sebum amount difference of skin after administrating the topical composition, wherein the abscissa is time(week) and the ordinate is the content of sebum(µ g/cm²).

### DESCRIPTION OF THE PREFERRED EMBODIMENTS

This invention provides a topical composition for transdermal administration, free of Vitamin A Acid, which is used for caring of skin and can be used to treat and improve the symptoms of the acne, comedo and zit of the skin.

The topical composition according to the present invention mainly comprise vitamin C, vitamin B complex, carotene, vitamin E, Flavors, thickening agent and surfactants. The main components of the composition are the essential Vitamins, but free of Vitamin A Acid, so there is no such problem such as over-dosage even applied over a long term. The need of vitamins is very small in the body, but the function of vitamins is very vital. Vitamins can't be synthesized by the body and needed to be ingested from outside. The major functions of vitamin C in the body are as followings: preventing the formation of peroxylipid, facilitating the formation of collagen, assisting many kinds of ferment, retarding the aging of cells, as well improving the blood circulation and the melanin reducing gradually. It is commonly considered that the vitamin C contributes to skin's regeneration, prevents the generation of melanin and enhances the immunity.

Vitamin B complex is a combination of various vitamins, such as vitamin B₁(thiamin), vitamin B₂(riboflavin), Niacin, vitamin B₅(pantothenic acid), vitamin B₆(pyridoxal), folic acid, vitamin B₁₂(cobalamin) and biotin etc. The main component is coenzyme, whose function is to facilitate the oxidation of glucose, improve fats and proteins releasing energy so as to keep the normal function of nervous system. They are necessary to the growth and regeneration of cells, the generation of erythrocyte, the synthesis of nucleoprotein and myelin, and it can also activate the folic acid-assisted ferment so as to facilitate the generation of erythrocyte.

Vitamin E is considered as oxidation inhibitor, which can inhibit the agglutination of blood platelet. Vitamin E can prevent the oxidation of erythrocyte cytomembrane so as to protect it from being destroyed which will cause anemia, it can also keep the integrity of cell membrane so as to enhance the function of linolenic acid, protect the structure and function of muscles and nerve tissues, and thus increase the blood flow at terminal vessel, and improve the situation of the blood flow. Although Vitamin E has the above-described activities, none of the commercially available products has the function of maintaining skin, the therapeutic efficacy for the acne, comedo, zit, or the function of antioxidation, such product includes those containing vitamin E and Wal Green or vitamin E-containing L'oreal Furtur E. The activity test demonstrates that the composition having the main components according to the present invention shows excellent efficacy. It should be noted that it can not be presumed from the conventional technology in the art, although the composition according to the present invention is consisted of vitamin C, vitamin B complex, carotin, vitamin E, flavor, thickening agent and surfactants.

The composition of the invention mainly comprise vitamin C 1-45%W/W, vitamin B complex 1-5%W/W, carotin 1-3%W/W, vitamin E 2-90%W/W, flavor 0.1-2% W/W, thickening agent 1-5%W/W, surfactants 1-8%W/W and appropriate amount of water. The preferred ratio of the main components is vitamin C 4-15%W/W, vitamin B complex 1-3% W/W, carotin 1-2%W/W and vitamin E 20-65%W/W.

This invention provides a Vitamin A Acid acid-free composition, the composition is useful in treatment acne, comedo, zit by locally apply. The composition comprise vitamin C, vitamin B complex, carotin, vitamin E, flavors, thickening agent and surfactants, and consequently can be applied onto the local area of the body, such as the skin and face, which will achieve the aim of nourishing skin, treating and improving of acne, comedo and zit, and protecting the skin from oxidation.

According to the present topical composition, various kind Excipients, carriers or diluting agent may be included, if necessary, and thus formulated as an ointment, emulsion, lotion or patch which could apply to affected site. Adhesives such as starch, sodium carboxymethylcellulose etc., are added to those dosage forms by conventional technique in the art. Buffer salt such as phosphate is also added thereto so as to adjust the pH value to proper range. Permeation enhancers or extracts from natural plants such as licorice root may be added thereto.

### Example 1

| Formulation | Lo-108 |
|---|---|
| Vitamin E | 65 |
| Vitamin C | 4 |
| Vitamin B complex | 1 |
| Carotene | 1 |
| Flavor | 1 |
| Surfactant | 6.5 |
| Thickening agent | 4 |
| Distilled water | 30 |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Example 2

| Formulation | Lo-110 |
|---|---|
| Vitamin E | 20 |
| Vitamin C | 20 |
| Vitamin B complex | 2 |
| Carotene | 2 |
| Flavor | 2 |
| Surfactant | 8 |
| Thickening agent | 1 |
| Distilled water | 20 |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Example 3

| Formulation | Lo-122 |
|---|---|
| Vitamin E | 85 |
| Vitamin C | 2 |
| Vitamin B complex | 1 |
| Carotene | 1 |
| Flavor | 0.1 |
| Surfactant | 3 |
| Thickening agent | 2 |
| Permeation enhancer | 0.5 |
| Distilled water | 15 |

Vitamin C and vitamin B complex were dissolved in a small amount distilled water; while carotene, vitamin E, flavor, surfactant and thickening agent were mixed together, and then this two solution were mixed together and then a predetermined amount of distilled water was added thereinto.

### Example 4

| Formulation | Lo-130 |
|---|---|
| Vitamin E | 40 |
| Vitamin C | 10 |
| Vitamin B complex | 3 |
| Carotene | 2 |
| Flavor | 2 |
| Surfactant | 10 |
| Thickening agent | 5 |
| Distilled water | 20 |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Example 5

| Formulation | Lo-18 |
|---|---|
| Vitamin E | 90 |
| Vitamin C | 1 |
| Vitamin B complex | 1 |
| Carotene | 1 |
| Flavor | 0.5 |
| Surfactant | 3 |
| Thickening agent | 3 |
| Distilled water | qs |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Example 6

| Formulation | Lo-27 |
|---|---|
| Vitamin E | 30 |
| Vitamin C | 45 |
| Vitamin B complex | 1 |
| Carotene | 2 |
| Flavor | 2 |
| Surfactant | 8 |
| Thickening agent | 1 |
| Permeation enhancer | 0.1 |
| Distilled water | qs |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Example 7

| Formulation | Lo-22 |
|---|---|
| Vitamin E | 80 |
| Vitamin C | 8 |
| Vitamin B complex | 4 |
| Carotene | 3 |
| Flavor | 0.1 |
| Surfactant | 3 |
| Thickening agent | 2 |
| Distilled water | qs |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Example 8

| Formulation | Lo-39 |
|---|---|
| Vitamin E | 30 |
| Vitamin C | 10 |
| Vitamin B complex | 5 |
| Carotene | 2 |
| Flavor | 2 |
| Surfactant | 10 |
| Thickening agent | 5 |
| Permeation enhancer | 1.5 |
| Distilled water | qs |

Vitamin C and vitamin B complex were dissolved in a small amount distilled water, while carotene, vitamin E, flavor, surfactant and thickening agent were mixed together, and then this two solution were mixed together and then a predetermined amount of distilled water was added thereinto.

### Activity Test:

### Test 1: the therapeutic efficacy evaluation for the acne patient administrated Lo-108

Material and method: An open clinical efficacy evaluation method is used in the study. Sixty patients suffering from acne had been selected as subjects during Feb to Aug in 2001 from the outpatients, including 30 male and 30 female. Their ages are between 17-42 and the average age is 25. Lo-108 will be used over six months by local application, and it will be applied to the surface for 3 to 8 hours each days(overnight). The patients will return visit once two weeks to record the number and properties changes, and the methods are as following:
1. The affected part is washed and cleared with washer milk(soap), and then the drug is applied to the dried affected part.
2. The dosage for application is about 2 ml, and then it is covered with bandage.
3. The drug is washed off after it has been administrated for 3 hours, and no emulsion (cream) to keep wet should be used after washing.
4. If there are pustules, it should be squeezed before the drug is applied.

The patients having pustules need to be administrated orally antibiotics, such as tetracycline or vibramycin for a week, other therapies for acne are prohibited in the rest time, such as Vitamin A Acid for oral admintration or topical use, hormone therapy.

The result is evaluated by the doctor and patients according to the therapy ratio and degree which is scaled by the number of acne, papules and pustule, and the degree of the red swelling of the skin.

**Table 1. The results of therapy evaluation for Lo-108**

| | Before applied (number) | After applied over 8 weeks (number) |
|---|---|---|
| mean no. of comedones | 43.5 | 20.1(p<0.01) |
| mean no. of papules | 21.0 | 2.1(p<0.001) |
| mean no. of pustules | 8.9 | 0(p<0.0001) |
| mean no. of cysts | 0.8 | 0 |

The results in table 1 shows, after applied over 8 weeks, the number of acne with black head or white head decreased significantly and the mean number changed from 43.5 to 20.1(p<0.01), the number of red papules with contracted pore-opening reduced significantly and changed from 21.0 to 2.1(p<0.001); the pustule and the red swelling of cyst disappeared and the mean number changed from 8.9 to 0, or from 0.8 to 0, or only some erythemas or depressed scars of were left over. During the evaluation, Lo-108 can not only eliminate inflammation, reduce the temperature and weaken swelling. but also can inhibit the hyperplasia tendency of hyperkeratosis of skin, thus make the skin more smooth. The improvement of depressed scars usually followed along with the amelioration of swelling, and this is probably due to detumescence.

The topical medicines for de-keratinization or antibiotics in the art can cause many side effects, such as dryness, peel, smartingand even red swlling, but these side effects did not appear after the Lo-108 is administrated because it's moisturising function.

From the results listed above, we concluded that Lo-108 has therapeutic efficacy for the acne, it also have the function of de-keratinizing, reducing the excretion of sebum, killing bacterium, eliminating inflammation and increasing the water content in cutin.

### Test 2. comparative evaluation of the inhibition from sebum excretion

Twenty healthy volunteers take part in this clinical test and their age is from 18 to 55. One side of the skin on their forehead is administrated with Lo-108, another side is administrated with nothing, which will be use as the control. The Lo-108 is administrated each night as following: the Lo-108 is applied to the skin with dosage of 1 ml-2 ml, then it will be washed off three hours later. The course of treatment is 4 weeks. The amount of sebum on both sides of the forehead skin should be determined each week, and each time, the sebum should be determined at regular interval.

The test of sebum excretion amount is done by determining the amount of skin grease using the Sebometer 810 PC(Courage and Khazaka Ltd, Germany). Many variations can decrease to minimum, because the percentage difference of sebum is obtained by determining the sebum amount on both side of the forehead skin. The principle of determining sebum amount is as following: there is a opaque plastic substance, its thickness and area and are 0.1mm and 64mm² respectively. After it is depressed on skin for 30 seconds, the transparency will increase because it absorbs the sebum. There is a linear correlation between the transparency and the amount of sebum that has been aborbed. In another word, the amount of sebum is in direct proportion to the transparency, as described in table 2, and the data obtained from the photometer can be transmitted into mg/cm² by a formula.

**Table 2. The determination of skin grease**

| Time | The mount of grease on the left and right of forehead skin (µ g/cm²) | |
|---|---|---|
| 0 week | 88.15±58.21 | 93.85±57.32 |
| 1 week | 82.80±55.46 | 94±60.45 |
| 2 week | 88.00±42.60 | 112.55±57.61 |
| 3 week | 81.75±50.29 | 118.8±57.61 |
| 4 week | 61.15±37.37 | 99.95±46.40 |

Because the test groups and the control groups were tested on the left or right forehead skin of the same person respectively, the interference of temperature, moisture, the movement degree and sweating degree of the volunteer can be eliminated and neglected. The amount of grease on the forehead in the test group is significantly lower than that of the control group by the statistical method of ANOVA(Analysis of variance). The difference has significance in statistics(Pr>F .0.0001). The results are showed in table 1. There is a tendency of becoming significance for the mean difference in each week by the statistical method of ANOVA. From the results of the amount of sebum in Lo-108 group listed in table 3, it can be demonstrated that the change of the sebum amount have the statistically significant difference(pr>F 0.0001) after applied over one week. From the results listed in table 4, we conclude that the similar results will be obtained if the drug is administrated for a longer time and the value will not increase with time (pr>F 0.2854). The results listed above show that Lo-108 can effectively eliminate or depress the excretion of grease up to 12 hours, and it can temporarily decelerate the open amount of sebum (the major cause of the acne) from the sebaceous gland. This efficacy can be obtained in a short time. The efficacy of inhibiting sebum can be maintained at least 4 weeks if it is administrated daily.

**Table 3. The amount of sebum**

| Time (week) | Lo-108 Group | | Control group | |
|---|---|---|---|---|
| | Mean (µ g/cm²) | Standard deviation (SD) | Mean (µ g/cm²) | Standard deviation (SD) |
| 0 | 88.15 | 58.21 | 93.85 | 57.32 |
| 1 | 82.80 | 55.46 | 94.00 | 60.45 |
| 2 | 88.00 | 42.60 | 112.55 | 57.61 |
| 3 | 81.75 | 50.29 | 118.8 | 57.61 |
| 4 | 61.15 | 37.37 | 99.95 | 46.40 |

| | | | | |
|---|---|---|---|---|
| Note: treated group/Lo-108 is applied to the left side of the forehead for the 20 patients; control group/ base without drug is applied to the right side of the forehead for the 20 patients. | | | | |

**Table 4. The difference of sebum amount**

| Source | DF(degree of freedom) | Anova SS | Mean Square | F Value | Pr > F (probability >F) |
|---|---|---|---|---|---|
| No. of patient | 19 | 229013.20 | 12053.33 | 6.88 | 0.0001 |
| Treat | 1 | 27518.58 | 27518.58 | 15.71 | 0.0001 |
| Number of times of treat | 4 | 11278.55 | 2819.64 | 1.61 | 0.1740 |
| Treat * time | 4 | 8869.17 | 2217.29 | 1.27 | 0.2854 |

| | | | | | |
|---|---|---|---|---|---|
| Note:treat*time means the interaction between treat and time. | | | | | |

### Test 3. The experiment of burning and recovery of scar in animal.

The age of male rat used in this experiment is 8 weeks, the species is Wistar. These animals are fed in the experimental animal center in Chenggong University, which is the only qualified division according to the foreign animal culturing standard (SPF) in the south. The rats should be fed in a laboratory with air conditioning, the temperature should be 25±1°C, and all the animals can eat and drink freely.

### Burning Experiment:

The process of this experiment is done mainly according to the method which has been described by Kistler et al, and the self-controls is used in this experiment. The experiment process is as following:

Each rat is anesthetized by pentobarbital at the dosage of 65mg/kg, after the rats go to deep slumber, the back of rats is divided into four part with the area of 4 cm², and then hair on the back of each part is shaved using a razor. Then, a red-burn iron sheet (about 80-85°C) is put onto the four parts of back for about 10 seconds, which will cause scald on the back of rats. After these processes have been finished, the iron sheet is take off and the wound is simply sterilized and cleared with hydrogen peroxide solution (37%). Then, the four parts are treated separately; the control part is not administrated with drug, the other three parts are administrated with vitamin E, the base of the product and the test sample respectively, and the dosage is depended on covering all the wound area. After each part is spread with the sample, the wound is bandaged tightly to prevent bacterium from infecting the wound. The dressing change should be done and the wound should be observed daily at a regular interval. The proofs should be reserved by taking pictures for the wound on the same time daily. Seven days later, the rats are sacrificed, and removed the tissues of the four parts for the pathological section, and the pathology changes of the wound are evaluated by the Dr Li jincheng, who is a pathology specialist and work in the Xinguang Medical Center.

### Results

According to the recovery of burning wound, the inflammation has little change at the part which is treated with the product. The results show that there is little difference between the control part and the part treated with the base of product. There is no inflammation change at the part treated with the products.

### The experiment of the recovery of wound:

The age of male rat used in this experiment is also 8 weeks, the species is Wistar. According to the process described above, the back of the anesthetized rats is divided into four fixed parts, the area of each one is about 4cm², the rat hair of each part is cut using a razor. Then, a incision is made on each part of the back using a scalpel, the length of wound is about 1 cm and the wound should be deep enough to expose the muscle layer. Then, the wound is simply sterilized and cleared with hydrogen peroxide solution (37%). Similarly, other parts are treated respectively. The control part is administrated without drug, the other three parts are administrated with vitamin E, the base of the product and the test sample respectively, and the dosage is depended on covering all the wound area. After each part is spread with the sample, the wound is bandaged tightly to prevent bacterium from infecting the wound. The dressing change should be done and the wound should be observed daily at a regular interval. The proof should be reserved by taking photos for the wound on the same time of each day. In the end, the efficacy difference between groups is compared according to the days of recovery. The fewer days it need, the faster the rats recover.

### Results

According to the recovery of the wound, we can find that time of recovery is short if it is treated with the product, which is about 7.13+1.27 days (N=8); while in the control group, it is about 11.00+2.24 days (N=8); in the group which is treated with the base of product, it is about 10.13+1.62 days (N=8); there is no significantly difference (P>0.05) between the control group and the group which is treated with base of product.

### Test 4. Bacteriostasis Test

Three kinds of stains are used in this test, such as Staphylococcus aureus, Methicillin Restant(ATCC33591), Staphylococcus aureus) and propionibacterium acnes(ATCC6919). All of them is cultured respectively with proper medium, and used to analysis the Lo-110 with the concentration of 0.03µl/ml, 0.1 µl/ml, 0.3µl/ml, 1µl/ml, 3µl/ml, 10µl/ml, 30µl/ml and 100µl/ml, the results shows that the inhibitory concentration is 100µl/ml.

Although the foregoing invention has been described in some detail by way of perfered embodiments, it will be obvious to those skilled in the art that the certain modifications and changes may be practiced within the scope of the invention.

## Claims

1. A topical composition for transdermal administration, wherein the main components comprise vitamin C 1~45%W/W, vitamin B complex 1~5%W/W, carotene 1~3%W/W, and vitamin E 2~90%W/W.

2. The topical composition according to claim 1, wherein the preferred percentage of main components comprise vitamin C 4~15% W/W, vitamin B complex 1~3%W/W, carotene 1~2%W/W, and vitamin E 20~65% W/W.

3. The topical composition according to claim 1 or 2, wherein further comprise flavor 0.1~2% W/W, thickening agent 1~5%W/W, surfactant 1~8% W/W and appropriate amount of distilled water.

4. The topical composition according to claim 1, wherein the composition is applied to local area of the skin.

5. A topical composition for transdermal administration, said composition is used for the manufacture of medicine useful in treatment of acne, comedo and zit, wherein mainly comprise vitamin C 1~45%W/W, vitamin B complex 1~5%W/W, carotene 1~3%W/W, vitamin E 2~90%W/W and flavor 0.1~2% W/W, thickening agent 1~5%W/W, surfactant 1~8%W/W and appropriate amount of distilled water.

6. A topical composition for transdermal administration, said composition is used for skin-care, wherein mainly comprise vitamin C 1~45%W/W, vitamin B complex 1~5%W/W, carotene 1~3%W/W, vitamin E 2~90%W/W and flavor 0.1~2%W/W, thickening agent 1~5%W/W, surfactant 1~8% W/W and appropriate amount of distilled water.

7. A topical composition for transdermal administration, said composition has an antioxidation property, wherein mainly comprise vitamin C 1~45%W/W, vitamin B complex 1~5%W/W, carotene 1~3%W/W, vitamin E 2~90%W/W and flavor 0.1~2%W/W, thickening agent 1~5% W/W, surfactant 1~8% W/W and appropriate amount of distilled water.

8. Use of a topical composition as defined in claim 1 in treatment of acne, comedo and zit.
